# EUROPEAN PATENT APPLICATION

(11) **EP 2 223 685 A1**
(43) Date of publication of application: **01.09.2010**
(21) Application number: 07817242.6
(22) Date of filing: 13.11.2007
(51) Int. Cl.: A61K 9/48, A61K 47/36, A61K 47/38, A61J 3/07

(54) **NON-GELATIN ENTERIC HARD CAPSULE SHELL AND PREPARATION METHOD THEREOF**

(71) Applicant: Shanghai Huiyuan Vegetal Capsule Co., Ltd, Shanghai 201203 (CN)
(72) Inventor: WU, Guoqing, Shanghai 201203 (CN); ZHANG, Shichu, Shanghai 201203 (CN)
(74) Representative: Kortekaas, Marcel C.J.A.
(86) International application number: PCT/CN2007/071052
(87) International publication number: WO 2009/062356

(57) **Abstract**

A composition for preparing non-gelatin enteric hard capsule shell comprises non-gelatin hydrophilic gel, enteric material, PH regulator, water, and optionally comprise coagulant aid(co-gelling agent), plasticizer and binder(adhesive). Said composition is substantially free of organic solvent. A preparation method of enteric hard capsule shell is disclosed.

## Description

### Field of the Invention

The present invention relates to material field, and more particularly to components for non-gelatin enteric hard capsule shell and the method of producing the same.

### Background of the Invention

At present, enteric materials are employed in the manufacture of enteric hard capsule shell. The exemplary enteric materials include shellac, polyvinyl acetate phthalate, acrylic resin, cellulose derivatives (which are, for example, cellulose acetate o-phthalate, cellulose acetate 1,2,4-benzenetricarboxylate, hydroxypropyl methyl cellulose phthalate, hydroxypropyl methyl cellulose 1,2,4-benzenetricarboxylate, cellulose acetate succinate, hydroxypropyl methylcellulose acetate succinate) and the like.

All of the enteric hard capsules using enteric materials mentioned above need a large amount of organic solvent, thus, easy to cause fire and explosion. Furthermore, the rates of its disintegration and dissolution are not satisfactory. The disadvantage of such an enteric hard capsule shell further includes that it is easy to be deformed or brittle at lower moisture content or at a higher or lower temperature, which is disadvantageous for manufacture of medicines. In addition, the common enteric hard capsule is made by double dipping technique, so the process for producing enteric hard capsule is complicated.

In addition to the disadvantage related to the manufacture and performance mentioned above, the enteric hard capsule of prior art also needs improvements on its composition. Generally, the film of enteric hard capsule is made from gelatin extracted from the materials such as bone or skin of cattle, porcine or the like, which is used to produce a gastric-soluble hard capsule blank, whereafter it is coated with a gelatin solution of an enteric material such as shellac, polyvinyl acetate phthalate, acrylic resin, cellulose derivative and the like in organic solvent by dipping or spraying to obtain a enteric hard capsule.

Although gelatin exhibits its advantage in producing the hard capsule, gelatin may be antigenic as an heterologous protein. For example, a cattle-derived gelatin material may contain a prion, causing the propagation of BSE if it is used for producing a soft capsule. Moreover, gelatin enteric hard capsule has other shortcomings, e.g. its strictly restricted tolerance to temperature and humidity, so its application is limited. Furthermore, gelatin enteric hard capsule is merely swollen in cold water and can't be dissolved in cold water. Gelatin enteric hard capsule shell tends to deform at higher moisture or temperature, and become brittle at lower temperature or with lower water content, and aldehyde drugs can't be encapsulated. Meanwhile its transparency is not satisfactory.

With the increasing demand for non-gelatin materials, it is urgent to develop a non-gelatin enteric hard capsule material and a method for producing the same.

To overcome the disadvantages of gelatin enteric hard capsule mentioned above, it is urgent to develop a new non-gelatin enteric hard capsule shell material and a method for producing the same, to provide an alternative for the gelatin enteric hard capsule shell material wherein, furthermore, the new non-gelatin enteric hard capsule shell material possesses good physical properties, and is produced without usage of organic solvent to simplify the process.

### Summary of the Invention

An object of the present invention is to obtain a composition for producing a non-gelatin enteric hard capsule shell material, which, with good properties, can replace the gelatin enteric hard capsule shell material and basically be free of organic solvent.

Another object of the present invention is to obtain a non-gelatin enteric hard capsule shell material, which, with good properties, can replace the gelatin enteric hard capsule shell material.

Another object of the present invention is to obtain a non-gelatin enteric hard capsule, which, with good properties, can replace the gelatin enteric hard capsule shell material.

Another object of the present invention is to obtain a method for producing a non-gelatin enteric hard capsule shell material, which can avoid using an organic solvent and making the process for manufacture simple.

In the first aspect according to the present invention, provided herein is a composition for a non-gelatin enteric hard capsule shell material comprising the following components by weight:
(A) 0.01∼18 parts by weight of non-gelatin hydrophilic gelling agent;
(B) 20∼97 parts by weight of an enteric material;
(C) 0.01∼25 parts by weight of a pH regulator;
(D) 40∼600 parts by weight of water;
(E) 0∼5 parts by weight of a co-gelling agent;
(F) 0∼5 parts by weight of a plasticizer;
(G) 0∼80 parts by weight of an adhesive;
and basically being free of organic solvent.

Preferably , the organic solvent is present in the composition in an amount no more than 5%wt, more preferably no more than 1%wt. Most preferably the composition is free of organic solvent.

In a specific embodiment according to the present invention, the composition comprises the following components by weight:
(A) 0.01∼18 parts by weight of a non-gelatin hydrophilic gelling agent;
(B) 30 parts by weight of an enteric material;
(C) 0.01∼25 parts by weight of a pH regulator;
(D) 40∼600 parts by weight of water;
(E) 0∼5 parts by weight of a co-gelling agent;
(F) 0∼5 parts by weight of a plasticizer;
(G) 0∼80 parts by weight of an adhesive;
and basically is free of organic solvent.

In a specific embodiment according to the present invention, the component(B) enteric material is selected from the group consisting of: polyvinyl acetate phthalate, acrylic resin, pullulan acetate, cellulose phthalate, cellulose acetate 1,2,4-benzenetricarboxylate, hydroxypropylmethyl cellulose phthalate, hydroxypropyl methyl cellulose 1,2,4-benzenetricarboxylate, cellulose acetate succinate and hydroxypropyl methylcellulose acetate succinate or combination thereof.

In a specific embodiment according to the present invention, the component (C) pH regulator is selected from the group consisting of: sodium carbonate, potassium carbonate, sodium hydroxide, potassium hydroxide, calcium hydroxide, ammonium hydroxide, sodium bicarbonate, potassium bicarbonate, ammonium carbonate, disodium hydrogen phosphate, dipotassium hydrogen phosphate, diammonium hydrogen phosphate, triethanolamine or combination thereof.

In the first aspect according to the present invention, provided herein is a non-gelatin enteric hard capsule shell material, comprising the following components:
(A) 0.01∼18 parts by weight of a non-gelatin hydrophilic gelling agent;
(B) 20∼97 parts by weight of an enteric material;
(C) 0.01∼25 parts by weight of a pH regulator;
(E) 0∼5 parts by weight of a co-gelling agent;
(F) 0∼5 parts by weight of a plasticizer;
(G) 0∼80 parts by weight of an adhesive;
wherein the total weight of the components (A), (B) and (C) is 85∼100%wt of the hard capsule shell material, and
wherein the hard capsule shell material further comprises 3∼12%wt water, based on the total weight of the hard capsule shell material.

In a specific embodiment according to the present invention, the hard capsule shell material comprises the following components:
(A) 0.01∼18 parts by weight of a non-gelatin hydrophilic gelling agent;
(B) 30 parts by weight of an enteric material;
(C) 0.01∼25 parts by weight of a pH regulator;
(E) 0∼5 parts by weight of a co-gelling agent;
(F) 0∼5 parts by weight of a plasticizer;
(G) 0∼80 parts by weight of an adhesive;
wherein the total weight of the components (A), (B) and (C) is 85∼100%wt of the hard capsule shell material, and
wherein the hard capsule shell material further comprises 3∼12%wt water, based on the total weight of the hard capsule shell material.

In a specific embodiment according to the present invention, the component (A) gelling agent is selected from the group consisting of: acetylated gellan gum, deacetylated gellan gum, κ-carrageenan, I-carrageenan. β-carrageenan, agar, pectin, sodium alginate, xanthan gum, tragacanth, karaya gum, locust bean gum, furcellaran, tamarind gum, tara gum, scleroglycan, microorganism alginate, carbomer or combination thereof.

In a specific embodiment according to the present invention, the component (B) enteric material is selected from the group consisting of: polyvinyl acetate phthalate, acrylic resin, pullulan acetate, cellulose acetate phthalate, cellulose acetate 1,2,4-benzenetricarboxylate, hydroxypropylmethyl cellulose phthalate, hydroxypropyl methyl cellulose 1,2,4-benzenetricarboxylate, cellulose acetate succinate and hydroxypropyl methylcellulose acetate succinate or combination thereof.

In a specific embodiment according to the present invention, the component (C) pH regulator is selected from the group consisting of: sodium carbonate, potassium carbonate, sodium hydroxide, potassium hydroxide, calcium hydroxide, ammonium hydroxide, sodium bicarbonate, potassium bicarbonate, ammonium carbonate, disodium hydrogen phosphate, dipotassium hydrogen phosphate, diammonium hydrogen phosphate, triethanolamine or combination thereof.

Preferably, the component (E) co-gelling agent is selected from the group consisting of: potassium chloride, calcium chloride, tripotassium phosphate, potassium citrate or combination thereof.

Preferably, the component (F) plasticizer is selected from the group consisting of: glycerin, sorbitol, polyethylene glycol, ethylene glycol , ethyl acetate , 1,3-butanediol , 1,4-butanediol, xylitol, glycerol biacetate, glycerol triacetate, glycerol monoacetate, mannitol, inositol, maltitol, glucose, polypropylene glycol or combination thereof.

Preferably, the component (G) adhesive is selected from starch, dextrin, polyvinylpyrrolidone, carboxymethyl chitin, polyvinyl alcohol, sesbania gum, acacia gum, cassia gum, pullulan, elsinan, india rubber, glucan, polyvinyl pyrolidone/vinyl acetate copolymer, hydroxyalkyl cellulose, carboxyl alkyl cellulose or combination thereof.

In a specific embodiment according to the present invention, the component (G) adhesive is selected from the group consisting of: high amylose starch, etherified starch, etherified high amylose starch, etherified high amylopectin starch, oxidized starch, oxidized high amylose starch, oxidized high amylopectin starch, esterfied starch, esterfied high amylose starch, esterfied high amylopectin starch or combination thereof.

In another aspect according to the present invention, provided herein, is a non-gelatin enteric hard capsule made from the non-gelatin enteric hard capsule shell material according to the present invention.

The present invention also provides a method for producing a non-gelatin enteric hard capsule shell material comprising the following steps:
(a) providing 0.01∼18 parts by weight of a non-gelatin hydrophilic gelling agent, 20∼97 parts by weight of an enteric material; 0.01∼25 parts by weight of a pH regulator, 0∼5 parts by weight of a co-gelling agent, 0∼5 parts by weight of a plasticizer, 0∼80 parts by weight of an adhesive and 40∼600 parts by weight of water, and blending them with heating to obtain a dissolved gel solution;
(b) producing a non-gelatin enteric hard capsule shell material with the gel solution of step (a) by single dipping technique or pressing technique at a temperature of 50∼60°C;
(c) drying the non-gelatin enteric hard capsule shell material of step (b) at a temperature of 30∼45°C, to form the non-gelatin enteric hard capsule shell material with 3∼12%wt water.

### Embodiments

Through the continuing research works of the inventors on the characteristics of non-gelatin materials, it was found that, after combining an adhesive, an hydrophilic gelling agent and an enteric material at a certain ratio and regulating pH of the gel solution with a pH regulator, the enteric hard capsule shell material and capsule shell thereof, with good taste and high transparency, may be produced without any organic solvent, to overcome the shortcomings of prior enteric hard capsule. The present invention was accomplished on the basis of this discovery.

The term "alkyl", as used herein, unless specified otherwise, refers to straight-chain or branched-chain hydrocarbon radicals containing 2∼20 carbon atoms, preferably, hydrocarbon radicals containing 1∼10 carbon atoms, e.g. an alkyl including but are not limited to methyl, ethyl, n-propyl, isopropyl, n-butyl, isobutyl and tert-butyl.

All aspects of the present invention are illustrated in detail as follows:
The present invention provides a composition for producing a non-gelatin enteric hard capsule shell material comprising the following components:
   (A) 0.01∼18 parts by weight of a non-gelatin hydrophilic gelling agent;
   (B) 20∼97 parts by weight of an enteric material;
   (C) 0.01∼25 parts by weight of a pH regulator;
   (D) 40∼600 parts by weight of water;
   (E) 0∼5 parts by weight of a co-gelling agent ;
   (F) 0∼5 parts by weight of a plasticizer ;
   (G) 0∼80 parts by weight of adhesive ;
   and basically being free of organic solvent.

Preferably, the organic solvent is present in the composition in an amount of no more than 5%wt based on the total weight of the composition, more preferably no more than 1%wt. Most preferably the composition is free of organic solvent.

The "organic solvent" according to the present invention includes alcohol, acetone, ether, hydrocarbon or combination thereof.

All components of the composition are described in detail as follows:

### (A) Non-gelatin hydrophilic gelling agent

There is no particular restriction on the constituents or molecular weight of the non-gelatin hydrophilic gelling agent according to the present invention, provided that the objects of the present invention are not restricted or limited hereby.

Representative examples of the non-gelatin hydrophilic gelling agent according to the present invention include, but are not limited to, acetylated gellan gum, deacetylated gellan gum, kappa carrageenan, iota carrageenan, β-carrageenan, agar, pectin, sodium alginate, xanthan gum, tragacanth, karaya gum, locust bean gum, furcellaran, tamarind gum, tara gum, scleroglycan, microorganism alginate, carbomer or combination thereof.

The acetylated gellan gum includes highly acetylated gellan gum and low acetylated gellan gum.

The highly acetylated gellan gum are commercially available, of which the degree of acetylation is common in the field. For example, the highly acetylated gellan gum refers to a gellan gum polysaccharide in which C6 of D-glucose residue in the repeat unit is substituted by acetyl by 50±10%, and is able to prevent gelling by intermolecular cross-linking, therefore having a good viscoelasticity and is called high acyl gellan gum.

The low acetylated gellan gum are commercially available, the degree of acetylation of which is common in the field. For example, the low acetylated gellan gum is a low acetyl gellan gum produced by deacetylation of a majority of acetyls with alkaline treatment under high temperature. Gellan gum of this type has good gelling property.

The microorganism in the microorganism alginate according to the present invention is a microorganism commonly used in the field, there is no restriction on it, provided that the microorganism alginate produced with it can be used as a hydrophilic gelling agent.

The hydrophilic gelling agent is generally present in the non-gelatin enteric hard capsule shell composition, the obtained non-gelatin enteric hard capsule shell material or non-gelatin enteric hard capsule shell according to the present invention in a amount of 0.01∼18 parts by weight, preferably 0.1∼18 parts by weight, more preferably 0.1∼8.5 parts by weight, while the content of the enteric material is 20∼97 parts by weight.

### (B) Enteric materials

The enteric materials according to the present invention are preferably polyvinyl acetate phthalate, acrylic resin, pullulan acetate, cellulose acetate phthalate, cellulose acetate 1,2,4-benzenetricarboxylate, hydroxypropylmethyl cellulose phthalate, hydroxypropyl methyl cellulose 1,2,4-benzenetricarboxylate, cellulose acetate succinate and hydroxypropyl methylcellulose acetate succinate or combination thereof.

The content of enteric material is typically 20∼97 parts, more preferably 30-80, more preferably 40∼60 parts, while the content of the hydrophilic gelling agent is typically 0.01∼18 parts by weight, preferably 0.1∼18 parts by weight, more preferably 0.1∼8.5 parts by weight.

### (C) pH regulator

The pH regulator according to the present invention is selected from : sodium carbonate, potassium carbonate, sodium hydroxide, potassium hydroxide, calcium hydroxide, ammonium hydroxide, sodium bicarbonate, potassium bicarbonate, ammonium carbonate, disodium hydrogen phosphate, dipotassium hydrogen phosphate, diammonium hydrogen phosphate, triethanolamine or combination thereof.

The content of the pH regulator is typically 0.01∼25 parts by weight, preferably 0.1∼8 parts by weight, while the content of the hydrophilic gelling agent is typically 0.01∼18 parts by weight, preferably 0.1∼18 parts by weight, more preferably 0.1∼8.5 parts by weight; the content of the hydrophilic gelling agent is typically 20∼97 parts by weight, preferably 30∼80 parts by weight, more preferably 40∼60 parts by weight.

The inventors have found that after adding a certain amount of pH regulator into the enteric material, the non-gelatin enteric hard capsule shell material can be produced without a large amount of organic solvent. In a preferred embodiment, after adding a certain amount of pH regulator into a specific enteric material, basically, there is no requirement for organic solvent, and a non-gelatin enteric hard capsule shell material can be produced by single dipping technique, meanwhile the disintegration rate of the obtained material is high, and the transparency is high, the specific enteric material is selected from polyvinyl acetate phthalate, acrylic resin, pullulan acetate, cellulose acetate phthalate, cellulose acetate 1,2,4-benzenetricarboxylate, hydroxypropylmethyl cellulose phthalate, hydroxypropyl methyl cellulose 1,2,4-benzenetricarboxylate, cellulose acetate succinate and hydroxypropyl methylcellulose acetate succinate or combination thereof.

### (D) Water

There is no particular restriction on water, provided that the objects of the present invention are not limited hereby. It can be, for example, purified water, deionized water, distilled water, mineral water or combination thereof.

The content of water in the non-gelatin plant-derived hard capsule shell is typically 40∼600 parts by weight, preferably 80∼400 parts by weight, while the content of the hydrophilic gelling agent is typically 0.01∼18 parts by weight, preferably 0.1∼18 parts by weight, more preferably 0.1∼8.5 parts by weight, and the content of the enteric material is typically 20∼97 parts by weight, preferably 30∼80 parts by weight, more preferably 40∼60 parts by weight.

### (E) Co-gelling agent

A co-gelling agent can be added into the hard capsule shell according to the present invention if needed.

Representative co-gelling agents include, but are not limited to, potassium chloride, calcium chloride, tripotassium phosphate, potassium citrate and combination thereof.

The content of the co-gelling agent is typically 0.01∼5 parts by weight, preferably 0.1∼4 parts by weight, while the content of hydrophilic gelling agent is typically 0.01∼18 parts by weight, preferably 0.1∼18 parts by weight, more preferably 0.1∼8.5 parts by weight.

In general, the content of the co-gelling agent varies inversely with the hydrophilic gelling agent.

### (F) Plasticizer

A plasticizer can be added into the hard capsule shell according to the present invention if needed.

Representative plasticizers include, but are not limited to, glycerin, sorbitol, polyethylene glycol and combination thereof, ethylene glycol, ethyl acetate, 1,3-butanediol, 1,4-butanediol, xylitol, glycerol biacetate, glycerol triacetate, glycerol monoacetate, mannitol, inositol, maltitol, glucose, polypropylene glycol or combination thereof.

The content of the plasticizer is typically 0.01∼5 parts by weight, preferably 0.1∼4 parts by weight, while the content of the hydrophilic gelling agent is typically 0.01∼18 parts by weight, preferably 0.1∼18 parts by weight, more preferably 0.1∼8.5 parts by weight.

Generally, the content of the plasticizer is related to the hardness of the final capsule, and varies with the hydrophilic gelling agent.

### (G) Adhesive

An adhesive can be added into the hard capsule shell according to the present invention if needed.

The adhesive according to the present invention is selected from starch, dextrin, polyvinylpyrrolidone, carboxymethyl chitin, polyvinyl alcohol, sesbania gum, acacia gum, cassia gum, pullulan, elsinan, india rubber, glucan, polyvinyl pyrolidone/vinyl acetate, hydroxyalkyl cellulose, carboxyl alkyl cellulose or combination thereof.

There is no particular restriction on the adhesive, provided that the objects of the present invention are not limited hereby.

There is no restriction on the polymerization process for and formulation of the copolymer, provided that the objects of the present invention are not limited hereby.

The cellulose, as used herein, includes the metallic salts thereof, for example, alkali metal salts, in particular, sodium salt, such as sodium carboxymethyl cellulose.

Preferably, the adhesive is selected from hydroxyalkyl cellulose, carboxyl alkyl cellulose, starch or combination thereof, in particular, for example, a combination of hydroxyalkyl cellulose and starch.

More preferably, the adhesive is starch.

Most preferably, the starch is selected from high amylose starch, etherified starch, etherified high amylose starch, etherified high amylopectin starch, oxidized starch, oxidized high amylose starch, oxidized high amylopectin starch, esterfied starch, esterfied high amylose starch, esterfied high amylopectin starch.

The content of the adhesive is typically 0∼80 parts by weight, preferably 0∼60 parts by weight, more preferably 0∼50 parts by weight, while the content of the hydrophilic gelling agent is typically 0.01∼18 parts by weight , preferably 0.1∼18 parts by weight, more preferably 0.1∼8.5 parts by weight, and the content of the enteric material is 20∼97 parts by weight.

It was found that the transparency of the hard capsule shell material produced by adding a specific starch as an adhesive into the composition is substantially increased, the specific starch is selected from high amylose starch, etherified starch, etherified high amylose starch, etherified high amylopectin starch, oxidized starch, oxidized high amylose starch, oxidized high amylopectin starch, esterfied starch, esterfied high amylose starch, esterfied high amylopectin starch.

Besides the components mentioned above, other additives can be added into the composition for the hard capsule shell material if needed, e.g., preservative, antioxidant, pigment, seasoning agent, flavoring agent, etc. There is no particular restriction on the constituents of other additives, provided that the objects of the present invention are not limited hereby. Representative preservatives include, but are not limited to, sodium benzoate, methyl p-hydroxybenzoate, propyl p-hydroxybenzoate and combination thereof. The content of the preservative is typically 0.001∼0.2%wt, preferably about 0.01∼0.19%, based on the total weight of the final hard capsule shell material. The content of the preservative varies with the adhesive in direct proportion.

Representative antioxidants include, but are not limited to, propyl gallate, tea polyphenol, phytic acid, phospholipid, L-ascorbic acid and combination thereof. The content of the antioxidant is typically 0.001∼0.2%wt, preferably about 0.01∼0.19%wt, based on the total weight of the final hard capsule shell material.

The present invention further provides a non-gelatin enteric hard capsule shell material comprising the following components:
(A) 0.01∼18 parts by weight of a non-gelatin hydrophilic gelling agent;
(B) 20∼97 parts by weight of an enteric material;
(C) 0.01∼25 parts by weight of a pH regulator;
(E) 0∼5 parts by weight of a co-gelling agent;
(F) 0∼5 parts by weight of a plasticizer;
(G) 0∼80 parts by weight of an adhesive,
wherein the total weight of the components (A),(B) and (C) is 85∼100%wt of the hard capsule shell material, and
wherein the hard capsule shell material further comprises 3∼12%wt water.

Among the components of the hard capsule shell material according to the present invention, (A) non-gelatin hydrophilic gelling agent; (B) enteric material; (C) pH regulator; (E) co-gelling agent; ( F) plasticizer; (G) adhesive are defined as above regarding to the components of the composition according to the present invention.

There is no particular restriction on water among the components of the hard capsule shell material according to the present invention, provided that the objects of the present invention are not limited hereby. It can be, for example, purified water, deionized water, distilled water, mineral water or combination thereof.

The content of water in the non-gelatin plant-derived hard capsule shell material is typically 3∼12%wt of the hard capsule shell material.

Other additives can be added into the hard capsule shell material if needed, the content of which is no more than 15%wt of the total weight of the hard capsule shell material. It can be, for example, preservative, antioxidant, pigment, seasoning agent, flavoring agent, etc. There is no particular restriction on the constituents of other additives, provided that the objects of the present invention are not limited hereby.

Representative preservatives include, but are not limited to, sodium benzoate, methyl p-hydroxybenzoate, propyl p-hydroxybenzoate and combination thereof. The content of the preservative is typically 0.001∼0.2%wt, preferably about 0.01∼0.19%, based on the total weight of the final hard capsule shell material. The content of the preservative varies with the adhesive.

Representative antioxidants include, but are not limited to, propyl gallate, tea polyphenol, phytic acid, phospholipid, L-ascorbic acid and combination thereof. The content of the antioxidant is typically 0.001∼0.2%wt, preferably about 0.01∼0.19%wt, based on the total weight of the final hard capsule shell material.

The method for producing a non-gelatin enteric hard capsule shell according to the present invention comprises the following steps:
(a) providing a non-gelatin hydrophilic gelling agent of 0.01∼18 parts by weight, a pH regulator of 0.01∼20 parts by weight, a enteric material of 20∼97 parts by weight, an optionally co-gelling agent of 0∼5 parts by weight, an optionally plasticizer of 0∼5 parts by weight, an adhesive of 0∼80 parts by weight, and combining them with water while heating to obtain a dissolved sol;
(b) producing a non-gelatin enteric hard capsule shell material with the sol from (a) by single dipping technique or pressing technique at 50∼60°C;
(c) drying the non-gelatin enteric hard capsule shell material from (b) at 30∼45°C, yielding the non-gelatin enteric hard capsule shell material with 3∼12%wt water.

There is no specific restriction on the heating temperature in step (a), provided that a dissolved sol can be obtained.

In a specific embodiment according to the present invention, a hydrophilic gelling agent, an adhesive with an enteric material were combined , then water and a pH regulator, a co-gelling agent and a plasticizer were added, subsequently, the mixture obtained was heated to obtain a non-gelatin enteric hard capsule shell material.

In a preferred example, firstly, a hydrophilic gelling agent of 1∼6 parts by weight, an adhesive of 5∼10 parts by weight, an enteric material of 10∼70 parts by weight, a pH regulator of 1∼15 parts by weight and a plasticizer of 3∼15 parts by weight were combined to obtain a mixture, then water of 28.8∼94 parts by weight was heated to 75∼95°C, subsequently the mixture is added into the water to obtain a liquid mixture; and an enteric hard capsule shell was produced by dipping technique. Upon being dried, the final capsule shell was obtained with about 3∼12%wt water.

The method of producing a non-gelatin enteric hard capsule shell according to the present invention can be performed on a now available device for producing the gelatin hard capsule shell. It is unnecessary to provide other devices, and the desired non-gelatin enteric hard capsule shell can be obtained by adjusting the drying temperature, humidity, coating thickness, operating speed, and model size, etc.

Compared with the prior art, the advantages of the present invention are as follows:
(1) the gel material for producing an enteric capsule shell is non-gelatin hydrophilic gelling agent, which is free of animal protein and fat, thus avoiding infection caused by animal prion;
(2) a plasticizer can be added if needed.
(3) a non-gelatin hydrophilic gelling agent is ready to dissolve, the swollen stage necessary for the current enteric capsule can be omitted, thus, the production process is time-saving.
(4) the tolerance of non-gelatin enteric hard capsule shell to temperature and humidity is beneficial to the production, storage and transportation of the capsule, while the poor tolerance of gelatin enteric hard capsule shell to temperature and humidity make its application limited.
(5) while remaining the advantages of gelatin enteric hard capsule shell, it overcomes the shortcomings of gelatin enteric hard capsule shell, for example, easy to be deformed under higher humidity or temperature, becoming brittle and the aldehyde drug cannot be encapsuled under low temperature or low humidity.
(6) a non-gelatin enteric hard capsule can dissolve even in the cold water, while a gelatin enteric hard capsule is swollen in the cold water and cannot dissolve.
(7) the present invention can produce a non-gelatin enteric hard capsule by single dipping technique,overcoming the shortcoming of producing a gelatin enteric hard capsule by double dipping technique.
(8) the present invention avoids the shortcoming of using a large amount of organic solvent as needed in producing a gelatin enteric hard capsule, thus, there is no risk of fire and explosion caused by organic solvent.

The chemicals provided by the present invention can be synthesized by commercially available starting materials and traditional chemical process.

Other aspects of the present invention are obvious for the skilled in the art from the disclosure herein.

The invention will be further illustrated with respect to the following examples. It will be appreciated that the example is only for illustration, the scope of the present invention is not intended to be limited thereto. The experiments without specific condition in the examples are performed according to the regular conditions, for example those listed in Beilstein Handbuch der Organische Chemie, chemical industry press,1996, or the condition suggested by the manufactory. Unless otherwise specified, the ratio and percent is based on weight.

Unless otherwise specified, all professional and science terms used herein have the meaning as well known to the skilled in the art. Furthermore, any methods and materials similar or equivalent to those disclosed herein can be used in the method according to the present invention.

### Example 1

**Non-gelatin enteric hard capsule shell material No.1**

| formulation | amount |
|---|---|
| acetylated gellan gum | 1.25g |
| hydroxypropyl methyl cellulose | 25g |
| hydroxypropyl methyl cellulose phthalate | 30g |
| calcium chloride | 1g |
| aqueous ammonia | 25ml (25g) |
| deionized water | 192.8g |

1.25g acetylated gellan gum, 30g hydroxypropyl methyl cellulose phthalate, 25ml aqueous ammonia, 1 g calcium chloride were added into 192.8g deionized water, then the mixture was heated to 100°C. After a uniform mixture was obtained, 25g hydroxypropyl methyl cellulose was added into it while stirring. Upon the temperature was maintained for a period of time and deforming, a hard capsule shell was produced by single dipping technique , which is then dried at 40°C. The water content of the obtained capsule shell is 2g (3%wt). The obtained non-gelatin enteric hard capsule shell is transparent, elastic and has good taste.

The disintegration time(opening time) is determined of 45 minutes according to the standard of disintegration for enteric hard capsule in PHARMACOPOEIA OF THE PEOPLES REPUBLIC OF CHINA, 2005.

### Example 2

**Non-gelatin enteric hard capsule shell material No.2**

| formulation | amount |
|---|---|
| iota carrageenan | 4g |
| hydroxypropyl starch | 16g |
| cellulose acetate 1,2,4-benzenetricarboxylate | 30g |
| calcium chloride | 2g |
| deionized water | 138g |
| sodium carbonate | 10g |

4g iota carrageenan, 16g hydroxypropyl starch, 2g calcium chloride, 30g cellulose acetate 1,2,4-benzenetricarboxylate, 10g sodium carbonate were added into 138g of deionized water, then the mixture was heated to 100°C. A uniform mixture was obtained. Upon the temperature was maintained for a period of time and deforming, a hard capsule shell was produced by single dipping technique, which is then dried at 40°C, and the water content of the obtained capsule shell is 4.8g (8%). The obtained non-gelatin enteric hard capsule shell is transparent, elastic and has good taste.

The disintegration time(opening time) is determined of 35 minutes according to the standard of disintegration for enteric hard capsule in PHARMACOPOEIA OF THE PEOPLES REPUBLIC OF CHINA, 2005.

### Example 3

**Non-gelatin enteric hard capsule shell material No.3**

| formulation | amount |
|---|---|
| hydroxypropyl methyl cellulose | 18g |
| kappa carrageenan | 1.6g |
| enteric acrylic resin | 30g |
| sodium hydroxide | 0.6g |
| potassium chloride | 0.24g |
| L- ascorbic acid | 0.12g |
| deionized water | 67.5g |
| Glycerin | 2.4g |

1.6g kappa carrageenan, 30g enteric acrylic resin, 2.4g glycerin, 0.24g potassium chloride, 0.12g L-ascorbic acid, 0.6g sodium hydroxide were added into 67.5g deionized water, then the mixture was heated to 90°C. After a uniform mixture is obtained, 18g hydroxypropyl methyl cellulose was added into it while stirring. Upon deforming and cooling down to 55°C, a hard capsule shell was produced by single dipping technique , which is then dried at 35°C, and the water content of the obtained capsule shell is 1.1g (3%).The obtained non-gelatin enteric hard capsule shell is transparent and elastic.

The disintegration time (opening time) is determined of 50 minutes according to the standard of disintegration for enteric hard capsule in PHARMACOPOEIA OF THE PEOPLES REPUBLIC OF CHINA, 2005.

### Comparative example 1

A gelatin capsule was produced according to the method as described in example 1, except that the plant-derived hydrophilic gelling agent and adhesive are replaced by the same amount of gelatin. Compared the obtained product-gelatin hard capsule with the products from example 1 and 2, the results are as follows:
(1) The storage performance of capsule is listed in table 1:

**Table 1 Comparison of storage performance of capsule**

| | | |
|---|---|---|
| Item | the non-gelatin hard capsules from examples 1 and 2 | hard gelatin capsule |
| T(°C) :0; relative humidity:50% | Not brittle | brittle |
| T(°C) :40; relative humidity:50% | Not deformed | deformed |

The storage period under the conditions listed above is one week.
(2)the transparency of the capsule is listed in table 2:

| | | | |
|---|---|---|---|
| item | the non-gelatin hard capsule from example 1 | The non-gelatin hard capsule from example 2 | hard gelatin capsule |
| transparency | relatively transparent | relatively transparent | light yellow |

The transparency was determined as follows:
5.0g sample was added into 90ml water, upon swollen, the sample was heated in 65∼70°C water bath to dissolve. After taking it out from the water bath, water was added to make the volume of the mixture to be 100ml. After being taken out 5ml and poured into 25ml Nessler glass(colorimeter cylinder), compared with a control of the same volume ( taking 30ml standard sodium chloride solution, pouring into 50ml measuring flask, adding nitric acid and silver nitrate solution, each 1 ml, adding water to 50 ml, standing for 5 minutes, regulating the color with caramel solution if needed) on the turbidity.
Each sample was tested for three times. If the turbidity of given sample in three tests is lower than the control, the result will be "relatively transparent".

Compared the turbidity of the three samples at the same time, the order of the turbidity is as follows:
The sample from example 2 >the sample from example 1 >the sample from comparative example 1.

All reference mentioned herein is incorporated by reference, as each is individually incorporated by reference. It is to be understand that variations and modifications may be made therein by the skilled in the art upon reading the teaching herein, which are within the spirit of the invention and the scope of the appended claims.

## Claims

1. A composition for producing a non-gelatin enteric hard capsule shell material, comprising the following components:
(A) 0.01∼18 parts by weight of a non-gelatin hydrophilic gelling agent;
(B) 20∼97 parts by weight of an enteric material;
(C) 0.01∼25 parts by weight of a pH regulator;
(D) 40∼600 parts by weight of water;
(E) 0∼5 parts by weight of a co-gelling agent;
(F) 0∼5 parts by weight of a plasticizer;
(G) 0∼80 parts by weight of an adhesive;
and being basically free of organic solvent.

2. The composition according to claim 1, wherein the component (B) enteric material is selected from the group consisting of: polyvinyl acetate phthalate, acrylic resin, pullulan acetate, cellulose acetate phthalate, cellulose acetate 1,2,4-benzenetricarboxylate, hydroxypropylmethyl cellulose phthalate, hydroxypropyl methyl cellulose 1,2,4-benzenetricarboxylate, cellulose acetate succinate and hydroxypropyl methylcellulose acetate succinate or combination thereof.

3. The composition according to claim 1, wherein the component (C) of pH regulator is selected from the group consisting of: sodium carbonate, potassium carbonate, sodium hydroxide, potassium hydroxide, calcium hydroxide, ammonium hydroxide, sodium bicarbonate, potassium bicarbonate, ammonium carbonate, disodium hydrogen phosphate, dipotassium hydrogen phosphate, diammonium hydrogen phosphate, triethanolamine or combination thereof.

4. A non-gelatin enteric hard capsule shell material, comprising the following components:
(A) 0.01∼18 parts by weight of a non-gelatin hydrophilic gelling agent;
(B) 20∼97 parts by weight of an enteric material;
(C) 0.01∼25 parts by weight of a pH regulator;
(E) 0∼5 parts by weight of a co-gelling agent;
(F) 0∼5 parts by weight of a plasticizer;
(G) 0∼80 parts by weight of an adhesive,
wherein the total weight of components (A), (B) and (C) is 85∼100% by weight of the hard capsule shell material; and
wherein said material further comprises 3∼12% by weight water.

5. The material according to claim 4, wherein, the component (A) gel is selected from the group consisting of: acetylated gellan gum, deacetylated gellan gum, kappa carrageenan, iota carrageenan, β-carrageenan, agar, pectin, sodium alginate, xanthan gum, tragacanth, karaya gum, locust bean gum, furcellaran, tamarind gum ,tara gum, scleroglycan, microorganism alginate, carbomer or combination thereof.

6. The material according to claim 4, wherein, the component (B) enteric material is selected from the group consisting of: polyvinyl acetate phthalate, acrylic resin, pullulan acetate, cellulose acetate phthalate, cellulose acetate 1,2,4-benzenetricarboxylate, hydroxypropylmethyl cellulose phthalate, hydroxypropyl methyl cellulose 1,2,4-benzenetricarboxylate, cellulose acetate succinate and hydroxypropyl methylcellulose acetate succinate or combination thereof.

7. The material according to claim 4, wherein, the component (C) pH regulator is selected from the group consisting of: sodium carbonate, potassium carbonate, sodium hydroxide, potassium hydroxide, calcium hydroxide, ammonium hydroxide, sodium bicarbonate, potassium bicarbonate, ammonium carbonate, disodium hydrogen phosphate, dipotassium hydrogen phosphate, diammonium hydrogen phosphate, triethanolamine or combination thereof.

8. The material according to claim 4,wherein, the component (G) adhesive is selected from the group consisting of: high amylose starch, etherified starch, etherified high amylose starch, etherified high amylopectin starch, oxidized starch, oxidized high amylose starch, oxidized high amylopectin starch, esterfied starch, esterfied high amylose starch, esterfied high amylopectin starch or combination thereof.

9. A non-gelatin enteric hard capsule , wherein, made from the non-gelatin enteric hard capsule shell material according to claim 2.

10. A method for producing a non-gelatin enteric hard capsule shell material, comprising the following steps:
(a) providing a non-gelatin hydrophilic gelling agent of 0.01∼18 parts by weight, an enteric material of 20∼97 parts by weight, a pH regulator of 0.01∼25 parts by weight, a co-gelling agent of 0∼5 parts by weight, a plasticizer of 0∼5 parts by weight, an adhesive of 0∼80 parts by weight and water of 40∼600 parts by weight, and combining them while heating to obtain a dissolved gelling solution;
(b) producing a non-gelatin enteric hard capsule shell material with the gelling solution from (a) by single dipping technique or pressing technique at 50∼60°C;
(c) drying the non-gelatin enteric hard capsule shell material from (b) at 30∼45°C, yielding the non-gelatin enteric hard capsule shell material with 3∼12%wt water.
